# EUROPEAN PATENT APPLICATION

(11) **EP 4 544 996 A1**
(43) Date of publication of application: **30.04.2025**
(21) Application number: 23857503.9
(22) Date of filing: 12.06.2023
(51) Int. Cl.: A61B 5/024, A61B 5/00

(54) **ELECTRONIC DEVICE FOR MEASURING BIOMETRIC INFORMATION AND OPERATION METHOD THEREOF**

(30) Priority: 25.08.2022 KR 20220107189; 15.09.2022 KR 20220116422
(71) Applicant: Samsung Electronics Co., Ltd., Suwon-si, Gyeonggi-do 16677 (KR)
(72) Inventor: KIM, Hyogil, Suwon-si Gyeonggi-do 16677 (KR); PARK, Jeongmin, Suwon-si Gyeonggi-do 16677 (KR); LEE, Yonghak, Suwon-si Gyeonggi-do 16677 (KR); JIN, Youngwoo, Suwon-si Gyeonggi-do 16677 (KR)
(74) Representative: Gulde & Partner
(86) International application number: PCT/KR2023/008022
(87) International publication number: WO 2024/043465

(57) **Abstract**

According to an embodiment, an electronic device may comprise a first sensor, a memory, and at least one processor, wherein the memory stores at least one instruction for causing, when executed by the at least one processor, the electronic device to: acquire, through the first sensor, a first biometric signal for measuring first biometric information of a user on the basis of a first configuration value; acquire first data of the first biometric information measured on the basis of the first biometric signal; when remeasurement of the first biometric information is requested, identify whether the remeasurement of the first biometric information is requested within a designated time from a time point of the acquisition of the first data; and on the basis of the identification that the remeasurement of the first biometric information is requested within the designated time from the time point of the acquisition of the first data, acquire, through the first sensor, a second biometric signal for measuring the first biometric information on the basis of a second configuration value different from the first configuration value. Various other embodiments are possible.

## Description

### [Technical Field]

Embodiments of the disclosure relate to an electronic device for measuring biometric information and an operation method thereof.

### [Background Art]

Recently, electronic devices including various sensors capable acquiring users' biometric signals have been developed.

For example, an electronic device may acquire photoplethysmograph (PPG) signals via a PPG sensor. The electronic device can provide the user with biometric information on at least one of pulse, saturation of peripheral oxygen (SpO₂), or blood pressure measured using the PPG signals.

### [Detailed Description of the Invention]

### [Technical Problem]

### [Technical Solution]

In accordance with an aspect of the disclosure, an electronic device may include a first sensor, a memory, and at least one processor.

The memory according to one embodiment may store at least one instruction that, when executed by the at least one processor, causes the electronic device to acquire a first biometric signal for measuring first biometric information of a user based on a first configuration value through the first sensor.

The memory according to one embodiment may store at least one instruction that, when executed by the at least one processor, causes the electronic device to acquire first data on the first biometric information measured based on the first biometric signal.

The memory according to one embodiment may store at least one instruction that, when executed by the at least one processor, causes the electronic device to determine whether re-measurement of the first biometric information is requested within a designated time from a time point of acquiring the first data when re-measurement of the first biometric information is requested.

The memory according to one embodiment may store at least one instruction that, when executed by the at least one processor, causes the electronic device to acquire a second biometric signal for measuring the first biometric information based on a second configuration value different from the first configuration value through the first sensor based on the determination that re-measurement of the first biometric information is requested within the designated time from the time point of acquiring the first data.

In accordance with another aspect of the disclosure, an operation method of an electronic device may include acquiring a first biometric signal for measuring first biometric information of a user based on a first configuration value through a first sensor included in the electronic device.

The operation method of the electronic device according to one embodiment may include acquiring first data on the first biometric information measured based on the first biometric signal.

The operation method of the electronic device may include determining whether re-measurement of the first biometric information is requested within a designated time from the time point of acquiring the first data when re-measurement of the first biometric information is requested.

The operation method of the electronic device may include acquiring a second biometric signal for measuring the first biometric information based on a second configuration value different from the first configuration value through the first sensor based on the determination that re-measurement of the first biometric information is requested within the designated time from the time point of acquiring the first data.

A non-transitory recording medium according to one embodiment may store at least one instruction capable of executing an operation of acquiring a first biometric signal for measuring first biometric information of a user based on a first configuration value through a first sensor included in an electronic device.

The non-transitory recording medium according to one embodiment may store at least one instruction capable of executing an operation of acquiring first data on the first biometric information measured based on the first biometric signal.

The non-transitory recording medium according to one embodiment may store at least one instruction capable of executing an operation of determining whether re-measurement of the first biometric information is requested within a designated time from the time point of acquiring the first data when re-measurement of the first biometric information is requested.

The non-transitory recording medium according to one embodiment may store at least one instruction capable of executing an operation of acquiring a second biometric signal for measuring the first biometric information based on a second configuration value different from the first configuration value through the first sensor based on the determination that re-measurement of the first biometric information is requested within the designated time from the time point of acquiring the first data.

### [Advantageous Effects]

### [Brief Description of Drawings]

FIG. 1 is a block diagram illustrating an electronic device in a network environment according to various embodiments.
FIG. 2A is a diagram illustrating an electronic device worn by a user according to one embodiment.
FIG. 2B is a schematic block diagram illustrating a configuration of an electronic device according to one embodiment.
FIG. 3 is a flowchart illustrating an operation method of an electronic device according to one embodiment.
FIG. 4 is a flowchart illustrating an operation method of an electronic device according to one embodiment.
FIG. 5 is a flowchart illustrating an operation method of an electronic device for describing a second configuration value different from a first configuration value according to one embodiment.
FIGS. 6A and 6B are flowcharts illustrating an operation method of an electronic device for describing a predetermined condition of FIG. 4 according to one embodiment.
FIGS. 7A, 7B, 7C, and 7D are diagrams illustrating a screen displayed by an electronic device according to one embodiment at the time of measuring biometric information.
FIG. 8 is a flowchart illustrating an operation method of an electronic device according to one embodiment.
FIGS. 9A and 9B are diagrams illustrating a case in which an electronic device according to one embodiment displays biometric information on a display.

### [Mode for Carrying out the Invention]

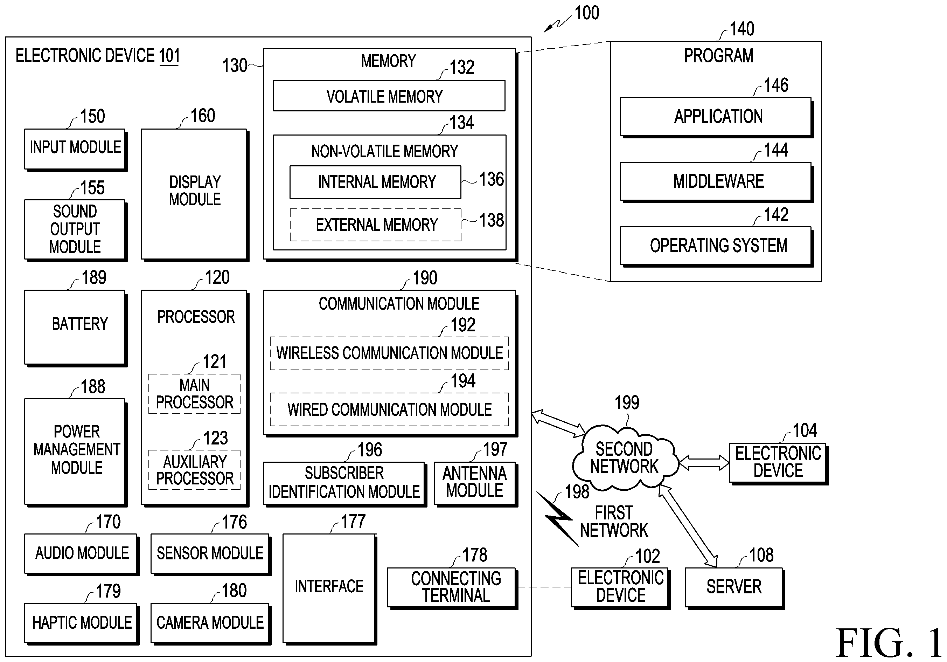

FIG. 1 is a block diagram illustrating an electronic device 101 in a network environment 100 according to various embodiments. Referring to FIG. 1, the electronic device 101 in the network environment 100 may communicate with an electronic device 102 via a first network 198 (e.g., a short-range wireless communication network), or at least one of an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input module 150, a sound output module 155, a display module 160, an audio module 170, a sensor module 176, an interface 177, a connecting terminal 178, a haptic module 179, a camera module 180, a power management module 188, a battery 189, a communication module 190, a subscriber identification module (SIM) 196, or an antenna module 197. In some embodiments, at least one of the components (e.g., the connecting terminal 178) may be omitted from the electronic device 101, or one or more other components may be added in the electronic device 101. In some embodiments, some of the components (e.g., the sensor module 176, the camera module 180, or the antenna module 197) may be implemented as a single component (e.g., the display module 160).

The processor 120 may execute, for example, software (e.g., a program 140) to control at least one other component (e.g., a hardware or software component) of the electronic device 101 coupled with the processor 120, and may perform various data processing or computation. According to one embodiment, as at least part of the data processing or computation, the processor 120 may store a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), or an auxiliary processor 123 (e.g., a graphics processing unit (GPU), a neural processing unit (NPU), an image signal processor (ISP), a sensor hub processor, or a communication processor (CP)) that is operable independently from, or in conjunction with, the main processor 121. For example, when the electronic device 101 includes the main processor 121 and the auxiliary processor 123, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one component (e.g., the display module 160, the sensor module 176, or the communication module 190) among the components of the electronic device 101, instead of the main processor 121 while the main processor 121 is in an inactive (e.g., sleep) state, or together with the main processor 121 while the main processor 121 is in an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123. According to an embodiment, the auxiliary processor 123 (e.g., the neural processing unit) may include a hardware structure specified for artificial intelligence model processing. An artificial intelligence model may be generated by machine learning. Such learning may be performed, e.g., by the electronic device 101 where the artificial intelligence is performed or via a separate server (e.g., the server 108). Learning algorithms may include, but are not limited to, e.g., supervised learning, unsupervised learning, semi-supervised learning, or reinforcement learning. The artificial intelligence model may include a plurality of artificial neural network layers. The artificial neural network may be a deep neural network (DNN), a convolutional neural network (CNN), a recurrent neural network (RNN), a restricted Boltzmann machine (RBM), a deep belief network (DBN), a bidirectional recurrent deep neural network (BRDNN), deep Q-network or a combination of two or more thereof but is not limited thereto. The artificial intelligence model may, additionally or alternatively, include a software structure other than the hardware structure.

The memory 130 may store various data used by at least one component (e.g., the processor 120 or the sensor module 176) of the electronic device 101. The various data may include, for example, software (e.g., the program 140) and input data or output data for a command related thereto. The memory 130 may include the volatile memory 132 or the non-volatile memory 134.

The program 140 may be stored in the memory 130 as software, and may include, for example, an operating system (OS) 142, middleware 144, or an application 146.

The input module 150 may receive a command or data to be used by another component (e.g., the processor 120) of the electronic device 101, from the outside (e.g., a user) of the electronic device 101. The input module 150 may include, for example, a microphone, a mouse, a keyboard, a key (e.g., a button), or a digital pen (e.g., a stylus pen).

The sound output module 155 may output sound signals to the outside of the electronic device 101. The sound output module 155 may include, for example, a speaker or a receiver. The speaker may be used for general purposes, such as playing multimedia or playing record. The receiver may be used for receiving incoming calls. According to an embodiment, the receiver may be implemented as separate from, or as part of the speaker.

The display module 160 may visually provide information to the outside (e.g., a user) of the electronic device 101. The display module 160 may include, for example, a display, a hologram device, or a projector and control circuitry to control a corresponding one of the display, hologram device, and projector. According to an embodiment, the display module 160 may include a touch sensor adapted to detect a touch, or a pressure sensor adapted to measure the intensity of force incurred by the touch.

The audio module 170 may convert a sound into an electrical signal and vice versa. According to an embodiment, the audio module 170 may obtain the sound via the input module 150, or output the sound via the sound output module 155 or a headphone of an external electronic device (e.g., an electronic device 102) directly (e.g., wiredly) or wirelessly coupled with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or a movement) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or a wireless communication channel between the electronic device 101 and the external electronic device (e.g., the electronic device 102, the electronic device 104, or the server 108) and performing communication via the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth^{™}, wireless-fidelity (Wi-Fi) direct, or infrared data association (IrDA)) or the second network 199 (e.g., a long-range communication network, such as a legacy cellular network, a 5G network, a next-generation communication network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The wireless communication module 192 may support a 5G network, after a 4G network, and next-generation communication technology, e.g., new radio (NR) access technology. The NR access technology may support enhanced mobile broadband (eMBB), massive machine type communications (mMTC), or ultra-reliable and low-latency communications (URLLC). The wireless communication module 192 may support a high-frequency band (e.g., the mmWave band) to achieve, e.g., a high data transmission rate. The wireless communication module 192 may support various technologies for securing performance on a high-frequency band, such as, e.g., beamforming, massive multiple-input and multiple-output (massive MIMO), full dimensional MIMO (FD-MIMO), array antenna, analog beam-forming, or large scale antenna. The wireless communication module 192 may support various requirements specified in the electronic device 101, an external electronic device (e.g., the electronic device 104), or a network system (e.g., the second network 199). According to an embodiment, the wireless communication module 192 may support a peak data rate (e.g., 20Gbps or more) for implementing eMBB, loss coverage (e.g., 164dB or less) for implementing mMTC, or U-plane latency (e.g., 0.5ms or less for each of downlink (DL) and uplink (UL), or a round trip of 1ms or less) for implementing URLLC.

The antenna module 197 may transmit or receive a signal or power to or from the outside (e.g., the external electronic device) of the electronic device 101. According to an embodiment, the antenna module 197 may include an antenna including a radiating element composed of a conductive material or a conductive pattern formed in or on a substrate (e.g., a printed circuit board (PCB)). According to an embodiment, the antenna module 197 may include a plurality of antennas (e.g., array antennas). In such a case, at least one antenna appropriate for a communication scheme used in the communication network, such as the first network 198 or the second network 199, may be selected, for example, by the communication module 190 (e.g., the wireless communication module 192) from the plurality of antennas. The signal or the power may then be transmitted or received between the communication module 190 and the external electronic device via the selected at least one antenna. According to an embodiment, another component (e.g., a radio frequency integrated circuit (RFIC)) other than the radiating element may be additionally formed as part of the antenna module 197.

According to various embodiments, the antenna module 197 may form a mmWave antenna module. According to an embodiment, the mmWave antenna module may include a printed circuit board, a RFIC disposed on a first surface (e.g., the bottom surface) of the printed circuit board, or adjacent to the first surface and capable of supporting a designated high-frequency band (e.g., the mmWave band), and a plurality of antennas (e.g., array antennas) disposed on a second surface (e.g., the top or a side surface) of the printed circuit board, or adjacent to the second surface and capable of transmitting or receiving signals of the designated high-frequency band.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. Each of the electronic devices 102 or 104 may be a device of a same type as, or a different type, from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, mobile edge computing (MEC), or client-server computing technology may be used, for example. The electronic device 101 may provide ultra low-latency services using, e.g., distributed computing or mobile edge computing. In another embodiment, the external electronic device 104 may include an internet-of-things (IoT) device. The server 108 may be an intelligent server using machine learning and/or a neural network. According to an embodiment, the external electronic device 104 or the server 108 may be included in the second network 199. The electronic device 101 may be applied to intelligent services (e.g., smart home, smart city, smart car, or healthcare) based on 5G communication technology or IoT-related technology.

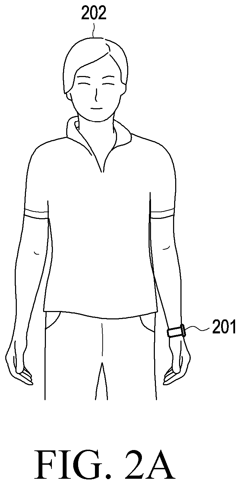

FIG. 2A is a diagram illustrating an electronic device worn by a user according to one embodiment.

Referring to FIG. 2A, according to one embodiment, an electronic device 201 can be worn by the user's body.

According to one embodiment, the electronic device 201 may be implemented as various forms of wearable electronic devices that can be worn by a user 202, such as a smart watch, a smart band, a smart ring, wireless earphones, or smart glasses. According to one embodiment, the electronic device 201 may be a device that can be worn on the wrist of the user 202 or a device that can be worn on another part of the human body (e.g., an arm, a head, a thigh, etc.). According to one embodiment, the electronic device 201 may be implemented as various forms of electronic devices (e.g., a smartphone) that can perform a communication function, as well as a wearable electronic device. According to one embodiment, the electronic device 201 may be implemented in the same or similar manner as the electronic device 101 of FIG. 1.

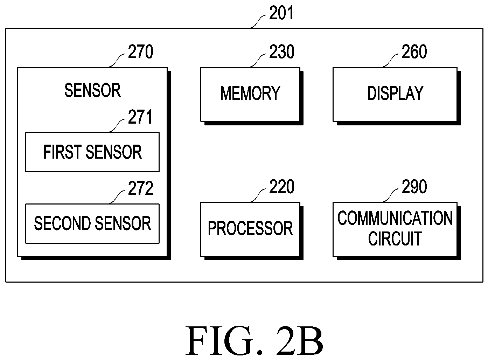

FIG. 2B is a schematic block diagram illustrating a configuration of an electronic device according to one embodiment.

According to one embodiment, the electronic device 201 (e.g., the electronic device 101 of FIG. 1) may include a processor 220, a memory 230, a display 260, a sensor 270, and a communication circuit 290.

According to one embodiment, the processor 220 may control the overall operation of the electronic device 201. For example, the processor 220 may be implemented in the same or similar manner as the processor 120 of FIG. 1.

According to one embodiment, the sensor 270 may include a first sensor 271 and/or a second sensor 272. For example, the sensor 270 may be implemented in the same or similar manner as the sensor module 176 of FIG. 1.

According to one embodiment, the processor 220 (e.g., the processor 120 of FIG. 1) may acquire a biometric signal for measuring the user's biometric information through the first sensor 271.

According to one embodiment, the processor 220 may execute a first application (e.g., application 146 of FIG. 1) for measuring the user's biometric information. The processor 220 may display an execution screen of the first application on the display 260.

According to one embodiment, the electronic device 201 may measure biometric information using the sensor 270 based on an input of the user 202 who requests biometric information measurement. Alternatively, according to one embodiment, the electronic device 201 may measure biometric information at predetermined designated time points based on an automatic measurement mode performed without an input from the user 202.

According to one embodiment, the processor 220 may display the measured biometric information on the display 260 (e.g., the display module 160 of FIG. 1).

According to one embodiment, the processor 220 may transmit the biometric information to an external electronic device through the communication circuit 290 (e.g., the communication module 190 of FIG. 1).

According to one embodiment, the first sensor 271 may include a photoplethysmography (PPG) sensor. According to one embodiment, the processor 220 may acquire or measure biometric information including heart rate, oxygen saturation, stress, arrhythmia, and/or blood pressure of the user 202 based on the biometric signal acquired using the first sensor 271.

According to one embodiment, the first sensor 271 may include a plurality of electrodes. The plurality of electrodes may be used to sense or detect a voltage corresponding to electrical resistance or a voltage corresponding to electrical conductivity by directly contacting the user's skin. According to one embodiment, the processor 220 may acquire a galvanic skin reflex (GSR), a bioelectrical impedance analysis (BIA) signal, and/or an electrocardiogram (ECG) signal using the first sensor 271. According to one embodiment, the processor 220 may measure biometric information including an electrocardiogram and/or body fat based on the biometric signal.

According to one embodiment, the processor 220 may use the first sensor 271 to determine the contact state of the user's 202 skin with respect to the electronic device 201. For example, the processor 220 may receive at least some of light reflected from the user's skin through the PPG sensor included in the first sensor 271 to determine the contact state of the skin of the user 202 with respect to the first sensor 271. Alternatively, the processor 220 may also determine the contact state of the skin of the user 202 with respect to the first sensor 271 through the plurality of electrodes included in the first sensor 271.

According to one embodiment, the second sensor 272 (e.g., the sensor module (176) of FIG. 1) may include at least one of a motion sensor, an infrared ray (IR) sensor, a temperature (body temperature) sensor, a gyro sensor, an acceleration sensor, a gravity sensor (or a geomagnetic sensor), or a barometer sensor. However, the present invention is not limited thereto, and the second sensor 272 may include various types of sensors capable of detecting the movement of the electronic device 201.

According to one embodiment, the processor 220 may determine whether the user 202 is in a state suitable for measuring biometric information using the second sensor 272. For example, the processor 220 may determine that the user 202 is not in the state suitable for measuring biometric information when it is determined that the movement of the user 202 is excessive compared to a designated condition. Alternatively, the processor 220 may determine that the user 202 is in the state suitable for measuring the biometric information when it is determined that the movement of the user 202 is not excessive compared to the designated condition.

According to one embodiment, the processor 220 may acquire a first biometric signal for measuring the first biometric information of the user 202 based on a first configuration value through the first sensor 271. For example, the first biometric signal may include a PPG signal. According to one embodiment, the processor 220 may acquire the first biometric signal by calculating a representative (e.g., maximum, minimum, median, or average) value of the biometric signal based on the biometric signal continuously (or discontinuously) acquired for a predetermined time. For example, the first configuration value may include a configuration value related to at least one of the light output intensity, wavelength, and driving frequency of the first sensor 271, or time (or cycle) required to acquire the biometric signal. However, the above-described configuration value is a simple example, and embodiments of the present invention may not be limited thereto.

According to one embodiment, the processor 220 may acquire first data on the first biometric information measured based on the first biometric signal. The first data may include at least one of a unique identification number of the first biometric information that is distinguished from other biometric information, a type of the first biometric information (e.g., heart rate, oxygen saturation, stress, arrhythmia, blood pressure, electrocardiogram, and/or body fat), a result value of the first biometric signal (e.g., heart rate per minute), a type of sensor used to acquire the first biometric signal (e.g., measurement using a PPG sensor or electrode), a time (or cycle) required to acquire the first biometric signal, a measurement type, a time point at which the first biometric signal is acquired, and a designated expiration time. According to one embodiment, the measurement type may mean information indicating whether a first biometric signal-acquisition request is a first measurement request or a re-measurement request that is not the first. According to one embodiment, the first data may be stored in the memory 230 (e.g., the memory 130 of FIG. 1) in a token unit. According to one embodiment, the token may mean a designated unit for storing data.

According to one embodiment, the processor 220 may store first data for the first biometric information in the memory 230.

According to one embodiment, when re-measurement of the first biometric information is requested, the processor 220 may determine whether the first data for the first biometric information has been previously acquired or stored. According to one embodiment, when it is determined that the first data for the first biometric information has not been acquired or stored, the processor 220 may acquire the first biometric signal for measuring the first biometric information of the user 202 based on the first configuration value of the first sensor 271.

According to one embodiment, when it is determined that the first data for the first biometric information has been acquired or stored, the processor 220 may determine whether re-measurement of the first biometric information is requested within a designated time (e.g., about 5 seconds) from a time point of acquiring the first data. According to one embodiment, the designated time may mean a designated expiration time included in the first data.

According to one embodiment, when it is determined that re-measurement of the first biometric information is requested within the designated time from the time point of acquiring or storing the first data, the processor 220 may acquire a second biometric signal for measuring the first biometric information of the user 202 based on a second configuration value that is different from the first configuration value. For example, the second biometric signal and the first biometric signal may be substantially the same type of biometric signals. For example, the second biometric signal may include a PPG signal. For example, the second configuration value may mean a configuration value of the first sensor 271 for measuring the biometric signal more accurately than when measuring the first biometric signal. Alternatively, when it is determined that re-measurement of the first biometric information is requested within the designated time from the time point of acquiring or storing the first data, the processor 220 may determine whether the user is in a state suitable for measuring biometric information. For example, the processor 220 may use the first sensor 271 to identify the contact state of the skin of the user 202 with the electronic device 201. Alternatively, the processor 220 may use the second sensor 272 to identify the movement of the electronic device 201. Alternatively, when re-measurement of the first biometric information is requested within the designated time from the time point of acquiring or storing the first data, the processor 220 may stop other operations of the electronic device 201. For example, the processor 220 may stop the execution of at least one application among other applications other than a first application (e.g., an application for measuring the user's biometric information) that may affect the measurement of the user's biometric information. Alternatively, the processor 220 may stop the transmission or reception operation of a signal that may affect the measurement of the user's biometric information.

According to one embodiment, when a sensing value acquired using the first sensor 271 and/or the second sensor 272 is within a predetermined range, the processor 220 may determine that a predetermined condition for measuring biometric information is satisfied. For example, the predetermined condition may mean a condition for determining whether the user is in a state suitable for measuring biometric information.

According to one embodiment, when it is determined that the predetermined condition is satisfied, the processor 220 may stop transmission or reception of a signal that may affect the acquisition of the biometric signal. Alternatively, the processor 220 may stop execution of at least one application other than the first application that may affect the acquisition of the biometric signal.

According to one embodiment, when it is determined that re-measurement of the first biometric information is requested after a predetermined time from the time point of acquiring the first data, the processor 220 may acquire a second biometric signal for measuring the first biometric information based on the first configuration value through the first sensor 271.

According to one embodiment, when re-measurement of the first biometric information is requested within a short time (or a predetermined time) from the time point of acquiring the first data, the electronic device 201 may re-measure the user's biometric information (e.g., oxygen saturation) using a relatively more accurately sensed biometric signal (e.g., PPG signal). Through this, the electronic device 201 may provide relatively more accurately measured biometric information so that the user does not have to repeatedly re-measure the biometric information.

The operations of the electronic device described below can be performed by the processor 220. However, for the convenience of description, the operations performed by the processor 220 will be described as being performed by the electronic device 201.

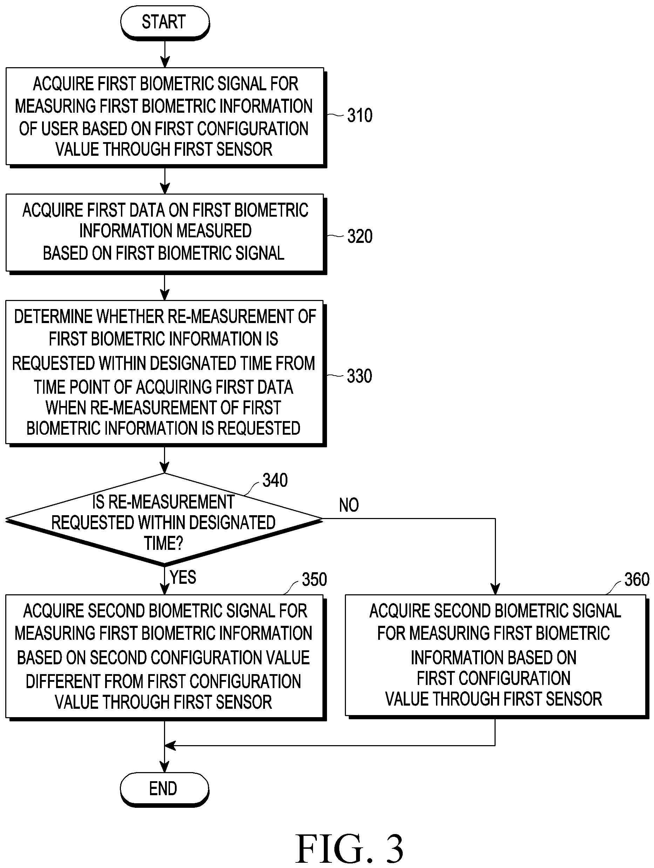

FIG. 3 is a flowchart illustrating an operation method of the electronic device 201 according to one embodiment.

In the following embodiment, respective operations may be performed sequentially, but are not necessarily performed sequentially. For example, the order of each operation may be changed, and at least two operations may be performed in parallel.

According to one embodiment, in operation 310, the electronic device 201 (e.g., the electronic device 201 of FIG. 2) may acquire a first biometric signal (e.g., a PPG signal) for measuring first biometric information (e.g., heart rate, oxygen saturation) of the user based on a first configuration value through the first sensor 271 (e.g., the first sensor 271 of FIG. 2).

According to one embodiment, in operation 320, the electronic device 201 may acquire or store first data for the first biometric information measured based on the first biometric signal. For example, the first data may include information on a designated time (e.g., an expiration time).

According to one embodiment, in operation 330, when re-measurement of the first biometric information is requested, the electronic device 201 may determine whether re-measurement of the first biometric information is requested within a designated time from the time point of acquiring the first data. For example, when re-measurement of the first biometric information is requested, the electronic device 201 may determine the first data (or information on the designated time included in the first data). The electronic device 201 may determine whether re-measurement of the first biometric information is requested within the designated time included in the first data.

According to one embodiment, when it is determined that re-measurement of the first biometric information is requested within the designated time (operation 340-YES), in operation 350, the electronic device 201 may acquire a second biometric signal for measuring the first biometric information based on a second configuration value different from the first configuration value through the first sensor 271. According to one embodiment, the electronic device 201 may acquire second data for re-measurement of the first biometric information based on the second configuration value. For example, the second configuration value may be a configuration value of the first sensor 271 for acquiring a biometric signal more accurately than the first configuration value. For example, the second configuration value may include a value for changing the sensing time of the first sensor 271, the driving frequency of the first sensor 271, and the light output intensity of the first sensor 271.

According to one embodiment, the electronic device 201 may acquire or sense the second biometric signal for a longer period of time than the acquisition or sensing time of the first biometric signal. Alternatively, according to one embodiment, the electronic device 201 may acquire the second biometric signal by operating the first sensor 271 at a driving frequency greater than the driving frequency of the first sensor for acquiring the first biometric signal.

Alternatively, according to one embodiment, the electronic device 201 may use light output with an intensity greater than that of light output from the first sensor used to acquire the first biometric signal, thereby acquiring the second biometric signal.

According to one embodiment, when it is determined that re-measurement of the first biometric information is requested after the designated time (operation 340-NO), in operation 360, the electronic device 201 may acquire a second biometric signal for measuring the first biometric information based on the first configuration value through the first sensor 271. For example, the electronic device 201 may acquire the second biometric signal under substantially the same condition as when the first data is acquired.

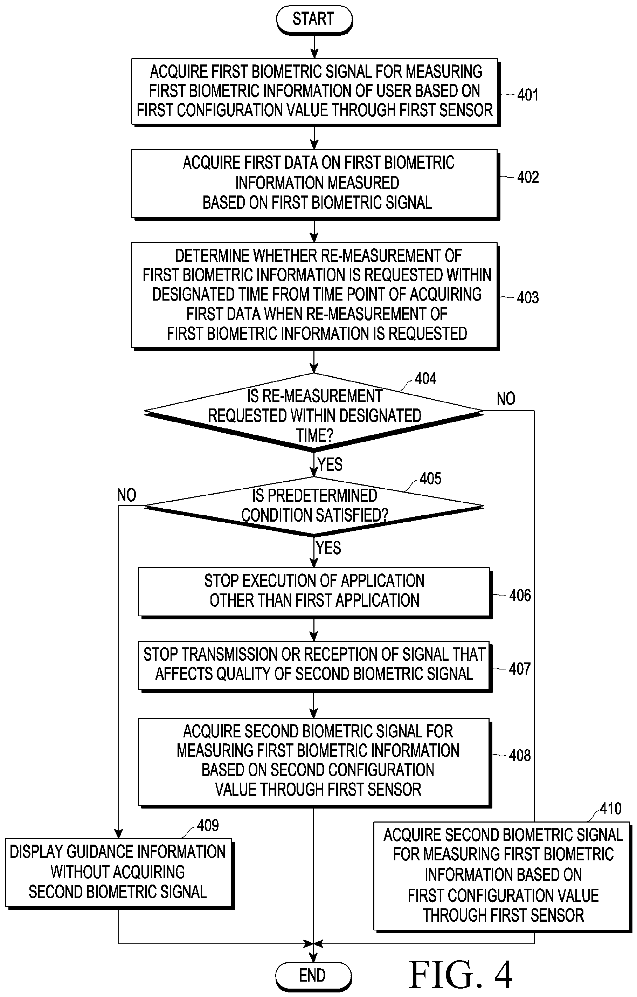

FIG. 4 is a flowchart illustrating an operation method of an electronic device according to one embodiment.

In the following embodiment, respective operations may be performed sequentially, but are not necessarily performed sequentially. For example, the order of each operation may be changed, and at least two operations may be performed in parallel.

According to one embodiment, in operation 401, the electronic device 201 may acquire a first biometric signal for measuring first biometric information of a user based on a first configuration value through the first sensor 271.

According to one embodiment, in operation 402, the electronic device 201 may acquire first data on the first biometric information measured based on the first biometric signal.

According to one embodiment, in operation 403, when re-measurement of the first biometric information is requested, the electronic device 201 may determine whether re-measurement of the first biometric information is requested within a designated time from the time point of acquiring the first data.

According to one embodiment, when it is determined that re-measurement of the first biometric information is requested after the designated time from the time point of acquiring the first data (operation 404-NO), in operation 410, the electronic device 201 may acquire a second biometric signal for measuring the first biometric information based on the first configuration value through the first sensor 271.

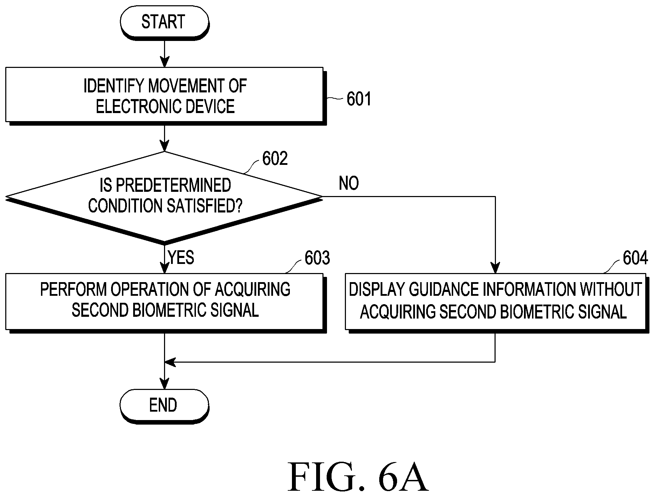

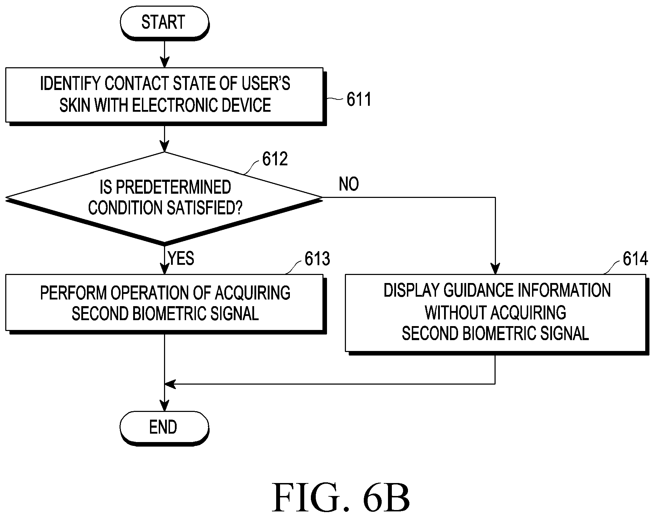

According to one embodiment, when it is determined that re-measurement of the first biometric information is requested within the designated time from the time point of acquiring the first data (operation 404-YES), in operation 405, the electronic device 201 may determine whether the electronic device 201 satisfies a predetermined condition. According to one embodiment, the predetermined condition may mean a condition indicating whether the electronic device 201 is in a state suitable for measuring biometric information from the user. For example, the predetermined condition may include a condition related to the movement of the electronic device 201 and/or the contact state of the user's skin with the electronic device 201. Meanwhile, specific embodiments for identifying whether the electronic device 201 satisfies the predetermined condition will be described in detail later with reference to FIGS. 6A and 6B.

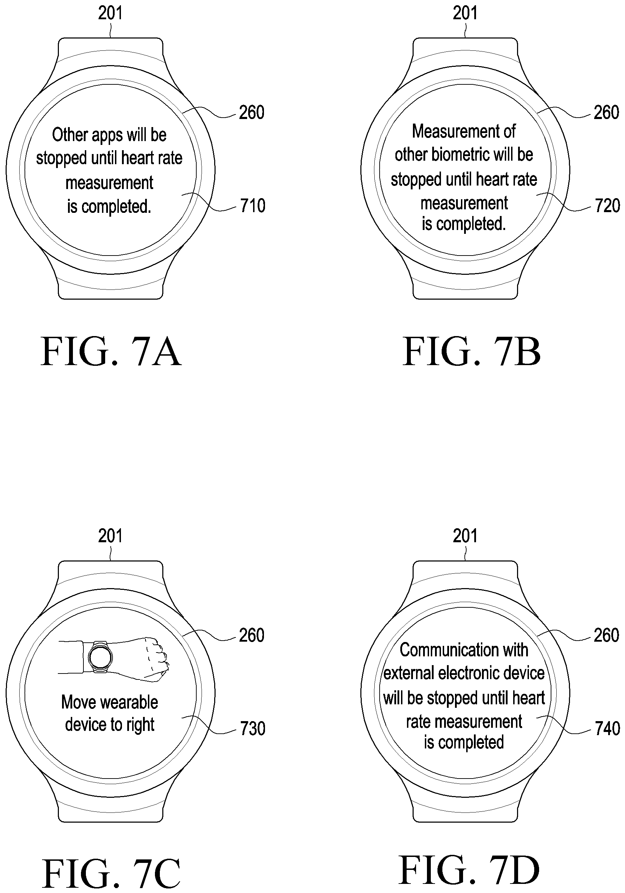

According to one embodiment, when it is determined that the electronic device 201 satisfies the predetermined condition (operation 405-YES), in operation 406, the electronic device 201 may stop the execution of at least one application among applications (e.g., a phone application, a message application, a camera application, etc.) other than a first application executed at the time of re-measurement of the first biometric information. According to one embodiment, the electronic device 201 may stop the execution of other applications until the second biometric signal for measuring the first biometric information is acquired. According to one embodiment, the electronic device 201 may display a message (e.g., 710 of FIG. 7A) indicating that execution of at least one application among applications other than the first application has been stopped, on the display 260.

According to one embodiment, when the electronic device 201 satisfies the predetermined condition, the electronic device 201 may stop measurement of at least one piece of biometric information among a plurality of pieces of biometric information (e.g., body fat information, oxygen saturation information, stress index information, etc.) other than the first biometric information at the time of re-measurement of the first biometric information (e.g., heart rate) when the predetermined condition is satisfied. According to one embodiment, the electronic device 201 may display a message (e.g., 720 of FIG. 7B ) indicating that measurement of at least one piece of biometric information among the plurality of pieces of biometric information other than the first biometric information has been stopped, on the display 260.

According to one embodiment, in operation 407, the electronic device 201 may stop transmission or reception of another signal (e.g., a wireless communication signal) that may affect the quality of the second biometric signal. According to one embodiment, the electronic device 201 may display a message (e.g., 740 of FIG. 7D) indicating that the transmission or reception of the other signal (e.g., wireless communication signal) has been stopped, on the display 260.

Although the electronic device 201 is described as performing operations 406 to 407, at least some of the operations may be omitted. Alternatively, according to one embodiment, the electronic device 201 may perform operations 406 and 407 in parallel. Alternatively, according to one embodiment, the order of operations 406 and 407 may be changed. According to one embodiment, the electronic device 201 may control some operations of the electronic device 201 that may affect acquisition of the biometric signal in addition to operations 406 and 407. This may be performed within a range that can be easily implemented by those skilled in the art.

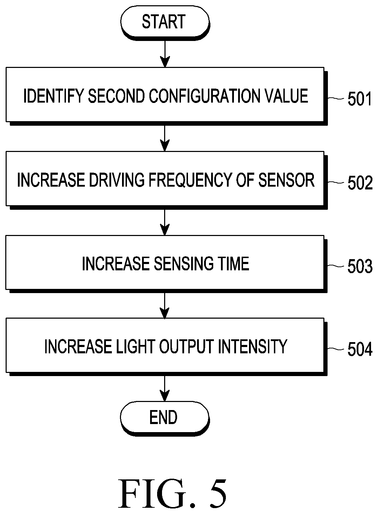

According to one embodiment, in operation 408, the electronic device 201 may acquire a second biometric signal for measuring the first biometric information based on a second configuration value that is different from the first configuration value through the first sensor 271. According to one embodiment, specific operations for acquiring the second biometric signal based on the second configuration value will be described in detail later in FIG. 5.

According to one embodiment, when the electronic device 201 does not satisfy the predetermined condition (operation 405-NO), in operation 409, the electronic device 201 may display guidance information on the display 260 without measuring the second biometric signal. According to one embodiment, the guidance information may include information related to the movement of the electronic device 201 or the contact state of the user's skin with the electronic device 201. According to one embodiment, the operation of displaying the guidance information is described in detail later in FIG. 6A and FIG. 6B.

FIG. 5 is a flowchart illustrating an operation method of an electronic device for describing a second configuration value different from a first configuration value according to one embodiment.

In the following embodiment, respective operations may be performed sequentially, but are not necessarily performed sequentially. For example, the order of each operation may be changed, and at least two operations may be performed in parallel.

According to one embodiment, in operation 501, the electronic device 201 may determine a second configuration value predetermined in the first sensor 271. According to one embodiment, the second configuration value may include a configuration value related to at least one of the light output intensity, wavelength, driving frequency, or sensing time (or cycle) required to acquire a biometric signal of the first sensor 271. However, the above-described configuration value is a simple example, and embodiments of the present invention may not be limited thereto.

According to one embodiment, in operation 502, based on the determined second configuration value, the electronic device 201 may increase the driving frequency of the first sensor 271. For example, the driving frequency based on the second configuration value may be greater than the driving frequency based on the first configuration value. Through this, the electronic device 201 may improve the accuracy and reliability of the second biometric signal acquired through the first sensor 271.

According to one embodiment, in operation 503, based on the determined second configuration value, the electronic device 201 may increase the sensing time of the first sensor 271. For example, the sensing time based on the second configuration value may be longer than the sensing time based on the first configuration value. Through this, the electronic device 201 may improve the accuracy and reliability of the second biometric signal acquired through the first sensor 271.

According to one embodiment, in operation 504, based on the determined second configuration value, the electronic device 201 may increase the light output intensity of the first sensor 271. For example, the light output intensity based on the second configuration value may be greater than the light output intensity based on the first configuration value. Through this, the electronic device 201 may be relatively less affected by the skin color, hairy skin, and/or tattoo of the user 202, and acquire more accurate biometric signals.

According to one embodiment, the electronic device 201 has been described as performing operations 502 to 504, but some of the operations may be omitted. Alternatively, according to one embodiment, the electronic device 201 may perform operations 502 to 504 in parallel. Alternatively, according to one embodiment, the order of operations 502 to 504 may be changed.

FIG. 6A is a flowchart illustrating an operation method of an electronic device for describing the predetermined condition of FIG. 4 according to one embodiment.

In the following embodiment, respective operations may be performed sequentially, but are not necessarily performed sequentially. For example, the order of each operation may be changed, and at least two operations may be performed in parallel.

According to one embodiment, in operation 601, the electronic device 201 may identify the movement of the electronic device 201. According to one embodiment, the electronic device 201 may identify the movement of the electronic device 201 using a second sensor 272 (e.g., an acceleration sensor, a gyro sensor).

According to one embodiment, in operation 602, the electronic device 201 may determine whether the movement of the electronic device 201 satisfies a predetermined condition. According to one embodiment, the electronic device 201 may determine that the predetermined condition is satisfied when the movement (e.g., acceleration value) of the electronic device 201 is less than or equal to a predetermined value. Alternatively, the electronic device 201 may determine that the predetermined condition is not satisfied when the movement (e.g., acceleration value) of the electronic device 201 exceeds the predetermined value.

According to one embodiment, the electronic device 201 may perform an operation of acquiring a second biometric signal for measuring the first biometric information in operation 603 when the predetermined condition is satisfied (operation 602-YES). According to one embodiment, the operation of acquiring the second biometric signal may include at least one of operations 406 to 408 of FIG. 4. For example, the operation of acquiring the second biosignal may include an operation of stopping execution of at least one application among applications other than a first application. For example, the operation of acquiring the second biometric signal may include an operation of stopping measurement of at least one piece of biometric information among a plurality of pieces of biometric information other than the first biometric information. For example, the operation of acquiring the second biometric signal may include an operation of stopping transmission or reception of a signal that may affect the quality of the second biometric signal. Alternatively, according to one embodiment, the operation of acquiring the second biometric signal may include an operation of acquiring a second biometric signal based on the second configuration value through the first sensor 271.

According to one embodiment, when the predetermined condition is not satisfied (operation 602-NO), the electronic device 201 may display guidance information on the display 260 without measuring the second biometric signal in operation 604. According to one embodiment, the electronic device 201 may display, on the display 260, a message (e.g., "Do not move while measuring biometric information") guiding the user to stop moving the electronic device 201. Alternatively, according to one embodiment, the electronic device 201 may output the guidance information as a voice. Alternatively, according to one embodiment, the electronic device 201 may output the guidance information as a predetermined vibration pattern.

FIG. 6B is a flowchart illustrating an operation method of an electronic device for describing the predetermined condition of FIG. 4 according to one embodiment.

In the following embodiment, respective operations may be performed sequentially, but are not necessarily performed sequentially. For example, the order of each operation may be changed, and at least two operations may be performed in parallel.

According to one embodiment, in operation 611, the electronic device 201 may identify the contact state of the user's skin with the electronic device 201. According to one embodiment, a biometric signal with noise may be acquired according to the contact state of the user's skin with the electronic device 201. According to one embodiment, the contact state may be identified based on a sensing value acquired using the first sensor 271. For example, the electronic device 201 may identify the contact state based on at least one of a current value, a voltage value, an impedance value, or a conductivity value sensed by the first sensor 271.

According to one embodiment, in operation 612, the electronic device 201 may determine whether the contact state of the user's skin with the electronic device 201 satisfies a predetermined condition. According to one embodiment, the electronic device 201 may determine that the predetermined condition is satisfied when the sensing value (e.g., PPG signal) received from the first sensor 271 is identified within a predetermined range (e.g., a measurable range of the first sensor 271) (e.g., a state in which the user's skin is in contact with the first sensor 271). Alternatively, the electronic device 201 may determine that the predetermined condition is not satisfied when the sensing value from the first sensor 271 is identified to be outside the predetermined range (e.g., when the user's skin is not in contact with the first sensor 271). Alternatively, the electronic device 201 may determine that the predetermined condition is not satisfied even when the sensing value is not identified by the first sensor 271.

According to one embodiment, the electronic device 201 may perform an operation of acquiring the second biometric signal for measuring the first biometric information in operation 613 when the predetermined condition is satisfied (operation 612-YES). According to one embodiment, the operation of acquiring the second biometric signal may include at least one of operations 406 to 408 of FIG. 4. For example, the operation of acquiring the second biometric signal may include an operation of stopping execution of at least one application among applications other than the first application. For example, the operation of acquiring the second biometric signal may include an operation of stopping measurement of at least one piece of biometric information among a plurality of pieces of biometric information other than the first biometric information. For example, the operation of acquiring the second biometric signal may include an operation of stopping transmission or reception of another signal that may affect the quality of the second biometric signal. Alternatively, according to one embodiment, the operation of acquiring the second biometric signal may include an operation of acquiring the second biometric signal based on the second configuration value through the first sensor 271.

According to one embodiment, when the electronic device 201 does not satisfy the predetermined condition (operation 612-NO), in operation 614, the electronic device 201 may display guidance information on the display 260 without measuring the second biometric signal. According to one embodiment, the electronic device 201 may display a message (e.g., 730 of FIG. 7C), on the display 260, for guiding the user to adjust the contact state of the user's skin with the electronic device 201. Alternatively, according to one embodiment, the electronic device 201 may output the guidance information as a voice. Alternatively, according to one embodiment, the electronic device 201 may output the guidance information as a predetermined vibration pattern.

FIGS. 7A, 7B, 7C, and 7D are diagrams illustrating a screen displayed by an electronic device according to one embodiment at the time of measuring biometric information.

FIG. 7A is a diagram illustrating a screen in which the electronic device 201 according to one embodiment displays, on the display 260, a message indicating that execution of at least one among applications other than a first application has been stopped.

Referring to FIG. 7A, according to one embodiment, the electronic device 201 may execute the first application and acquire a biometric signal for measuring or re-measuring user's biometric information. According to one embodiment, the electronic device 201 may stop the execution of applications other than the first application that is executed when measuring or re-measuring the user's biometric information.

According to one embodiment, the electronic device 201 may display, on the display 260, a first message 710 indicating that the execution of applications other than the first application has been stopped (e.g., "Execution of other apps will be stopped until heart rate measurement is completed").

According to one embodiment, the electronic device 201 may stop notifications (e.g., display on the display 260, sound, and/or vibration) from the other applications by stopping the execution of the other applications. As a result, the electronic device 201 may reduce changes in the psychological factors of the user 202 due to external influences, and measure more accurate biometric information.

FIG. 7B is a diagram illustrating a screen in which the electronic device 201 according to one embodiment displays, on the display 260, a message indicating that measurement of at least one piece of biometric information among a plurality of pieces of biometric information other than the first biometric information has been stopped at the time of measuring the first biometric information.

Referring to FIG. 7B, according to one embodiment, the electronic device 201 may measure or re-measure the first biometric information based on a user input requesting measurement of the first biometric information. According to one embodiment, the electronic device 201 may stop an automatic measurement mode in which biometric information is automatically measured at predetermined time intervals based on the user input requesting measurement of the first biometric information, and measure only the first biometric information.

According to one embodiment, the electronic device 201 may display, on the display 260, a second message 720 (e.g., "Measurement of other biometric information will be stopped until heart rate measurement is completed.") indicating that measurement of at least one piece of biometric information among a plurality of pieces of biometric information other than the first biometric information has been stopped at the time of measuring or re-measuring the first biometric information. In addition, although not illustrated, according to one embodiment, the electronic device 201 may stop the automatic measurement mode and display, on the display 260, a message (not shown) indicating that only the first biometric signal has been measured.

FIG. 7C is a diagram illustrating a screen in which the electronic device 201 according to one embodiment displays, on the display 260, a message for guiding the user 202 to adjust the contact state of the user's skin with the electronic device 201.

Referring to FIG. 7C, according to one embodiment, the electronic device 201 may identify the contact state between the electronic device 201 and the skin of the user 202 using the first sensor 271. According to one embodiment, the electronic device 201 may display, on the display 260, a third message 730 (e.g., "Move the wearable device to the right") that guides adjustment of the contact state of the skin of the user 202 with the electronic device 201 when a sensing value acquired using the first sensor 271 is out a predetermined range.

FIG. 7D is a diagram illustrating a screen in which the electronic device 201 according to one embodiment displays, on the display 260, a message notifying that communication with an external electronic device is stopped when measuring biometric information.

Referring to FIG. 7D, according to one embodiment, the electronic device 201 may stop transmitting or receiving a signal that may affect the quality of the biometric signal. For example, the electronic device 201 may stop a communication connection with an external electronic device (e.g., the electronic device 102 or 104 and the server 108 in FIG. 1) when measuring biometric information. According to one embodiment, the electronic device 201 may display, on the display 260, a fourth message notifying that communication with the external electronic device is stopped when measuring biometric information. The electronic device 201 may acquire a biometric signal by minimizing noise that may be generated due to a signal being transmitted or received.

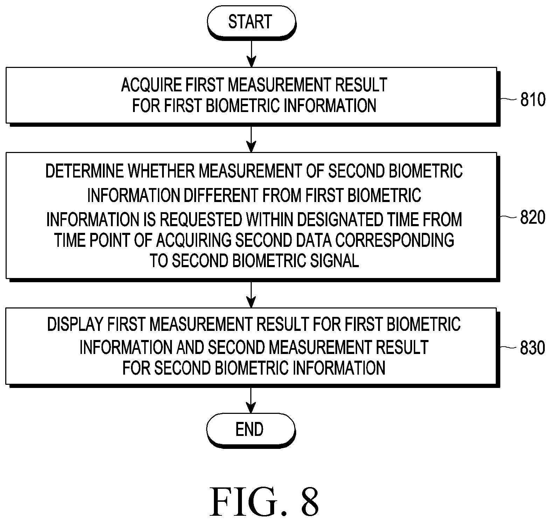

FIG. 8 is a flowchart illustrating an operation method of an electronic device according to one embodiment.

In the following embodiment, respective operations may be performed sequentially, but are not necessarily performed sequentially. For example, the order of each operation may be changed, and at least two operations may be performed in parallel.

According to one embodiment, in operation 810, the electronic device 201 (e.g., the electronic device 101 of FIG. 1) may acquire a first measurement result for the first biometric information. According to one embodiment, the first measurement result may include first biometric information (e.g., heart rate information) for a first biometric signal acquired based on a first configuration value and/or first biometric information (e.g., heart rate information) for a second biometric signal acquired based on a second configuration value.

According to one embodiment, in operation 820, the electronic device 201 may determine that measurement of second biometric information (e.g., oxygen saturation information) different from the first biometric information is requested within a designated time from the time point of acquiring second data corresponding to the second biometric signal. According to one embodiment, the second data may include data on the first biometric information for the second biometric signal acquired based on the second configuration value. For example, similarly to the first data, the second data may include at least one of a unique identification number, a type of the first biometric information (e.g., heart rate, oxygen saturation, stress, arrhythmia, blood pressure, electrocardiogram and/or body fat), a numerical result value of the first biometric information (e.g., heart rate 88 bpm), a type of sensor used to acquire the second biometric signal (e.g., PPG sensor, ECG sensor), a time (or cycle) required to acquire the second biometric signal, a measurement type (e.g., re-measurement), a time point at which the second biometric signal is acquired, or a specified expiration time.

According to one embodiment, the specified expiration time included in the second data may be configured to be a longer time than the specified expiration time included in the first data.

According to one embodiment, in operation 830, the electronic device 201 may display the first measurement result for the first biometric information and the second measurement result for the second biometric information on the display 260. According to one embodiment, the electronic device 201 may display the first measurement result including the first biometric information measured based on the first configuration value and/or the first biometric information measured based on the second configuration value together with the second measurement result for the second biometric information.

According to one embodiment, the electronic device 201 may display only the second measurement result for the second biometric information on the display 260 when it is determined that measurement of the second biometric information is requested after the designated time from the time point of acquiring the second data.

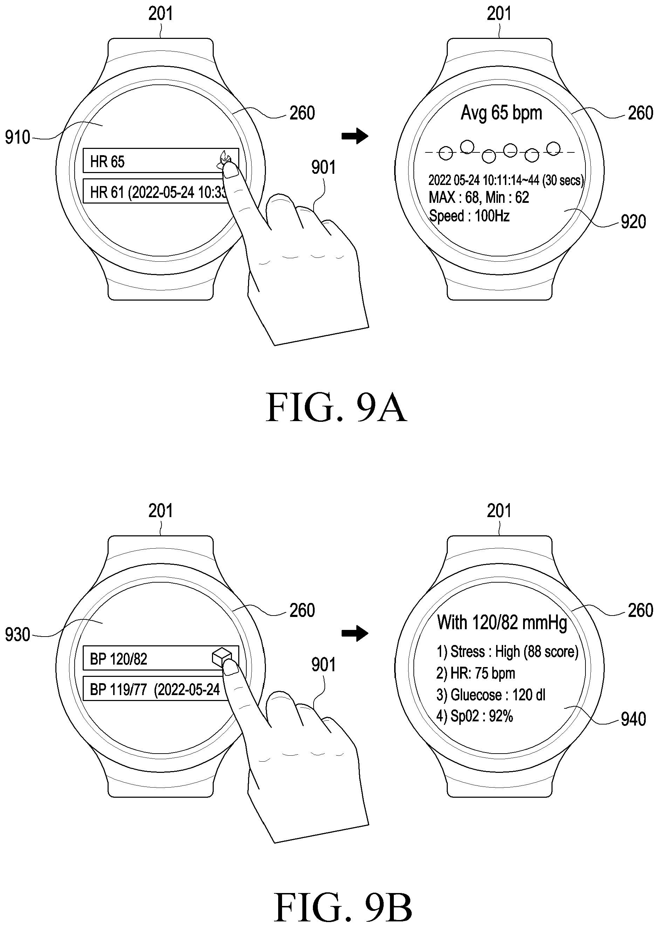

FIGS. 9A and 9B are diagrams illustrating a case in which the electronic device 201 according to one embodiment displays biometric information on the display 260.

Referring to FIG. 9A, the electronic device 201 according to one embodiment may display a first screen 910 including at least one piece of biometric information on the display 260. According to one embodiment, the at least one piece of biometric information may include data (e.g., first data) on a first biometric information measurement result or data (e.g., second data) on a re-measurement result for the first biometric information. According to one embodiment, the electronic device 201 may display at least one of the type of biometric information (e.g., heart rate {HR}), the numerical result value of biometric information (e.g., average heart rate per minute), and information on the time point of acquiring the biometric information or measurement type on the first screen 910.

According to one embodiment, the electronic device 201 may visually distinguish the first data and the second data and display this on the first screen 910. For example, the electronic device 201 may display the second data in a predetermined color or display a predetermined object only on the second data.

According to one embodiment, based on a user's input 901 of selecting a predetermined object, the electronic device 201 may display a second screen 920 including second data.

According to one embodiment, the second screen 920 may include at least one of an average heart rate per minute, a graph showing a heart rate measurement result, a heart rate measurement time, a maximum heart rate, a minimum heart rate, or a driving frequency of the first sensor 271. However, the above-described screens 910 and 920 are merely examples, and embodiments of the present invention may not be limited thereto.

Referring to FIG. 9B, the electronic device 201 according to one embodiment may display a screen including at least one piece of biometric information on the display 260.

According to one embodiment, the electronic device 201 may display a third screen 930 including first biometric information (e.g., blood pressure (BP), 119/77 mmHg) on the first biometric signal acquired based on the first configuration value, and first biometric information (e.g., BP, 120/82 mmHg) (e.g., the re-measurement result for the first biometric information) on the second biometric signal acquired based on the second configuration value.

According to one embodiment, based on a user's input (e.g., touch input) 901 of selecting a predetermined object, the electronic device 201 my display a fourth screen 940 including the re-measurement result (e.g., with 120/82 mmHg) for the first biometric information (e.g., BP information), second biometric information (e.g., stress index information), third biometric information (e.g., heart rate (HR) information), fourth biometric information (e.g., glucose information), and fifth biometric information (e.g., oxygen saturation (SpO₂) information). According to one embodiment, the biometric information may be listed in the order in which the biometric information was measured in time sequence (e.g., 1)->2)->3)->4)) and displayed on a fourth screen 940. However, the present invention is not limited thereto, and the biometric information may be listed and displayed in various ways.

According to one embodiment, the third data corresponding to the biometric signal acquired for measuring the second biometric information may have a longer designated expiration time than the second data. According to one embodiment, the fourth data corresponding to the biometric signal acquired for measuring the third biometric information may have a longer designated expiration time than the third data. Through this, the electronic device 201 may secure time for the processor 220 to analyze different biometric information, and display different biometric information together on the display 260.

According to one embodiment, the electronic device 201 may transmit the biometric information to an external electronic device through the communication circuit 290. According to one embodiment, the external electronic device may display the received biometric information on the display 260.

However, the screens 930 and 940 described above are merely examples, and embodiments of the present invention may not be limited thereto.

An electronic device according to one embodiment may include a first sensor, a memory, and at least one processor.

The memory according to one embodiment may store at least one instruction that, when executed by the at least one processor, causes the electronic device to acquire a first biometric signal for measuring first biometric information of a user based on a first configuration value through the first sensor.

The memory according to one embodiment may store at least one instruction that, when executed by the at least one processor, causes the electronic device to acquire first data on the first biometric information measured based on the first biometric signal.

The memory according to one embodiment may store at least one instruction that, when executed by the at least one processor, causes the electronic device to determine whether re-measurement of the first biometric information is requested within a designated time from the time point of acquiring the first data, when re-measurement of the first biometric information is requested.

The memory according to one embodiment may store at least one instruction that, when executed by the at least one processor, causes the electronic device to acquire a second biometric signal for measuring the first biometric information based on a second configuration value different from the first configuration value through the first sensor based on the determination that re-measurement of the first biometric information is requested within the designated time from the time point of acquiring the first data.

The electronic device according to one embodiment may further include a second sensor.

The at least one instruction according to one embodiment may cause, when executed by the at least one processor, the electronic device to identify a measurement state of the first biometric information using the first sensor or the second sensor.

The at least one instruction according to one embodiment may cause, when executed by the at least one processor, the electronic device to acquire the second biometric signal according to the identification result.

The at least one instruction according to one embodiment may cause, when executed by the at least one processor, the electronic device to identify a contact state of the user's skin with the electronic device using the first sensor.

The at least one instruction according to one embodiment may cause, when executed by the at least one processor, the electronic device to prevent the acquisition of the second biometric signal when the contact state of the user's skin does not satisfy a predetermined condition.

The at least one instruction according to one embodiment may cause, when executed by the at least one processor, the electronic device to identify a movement of the electronic device using the second sensor.

The at least one instruction according to one embodiment may cause, when executed by the at least one processor, the electronic device to prevent the acquisition of the second biometric signal when the movement of the electronic device does not satisfy a predetermined condition.

The at least one instruction according to one embodiment may cause, when executed by the at least one processor, the electronic device to stop transmission or reception of a signal that affects the quality of the second biometric signal based on the determination that re-measurement of the first biometric information is requested within the designated time from the time point of acquiring the first data.

The at least one instruction according to one embodiment may cause, when executed by the at least one processor, the electronic device to start re-measurement of the first biometric information of the user based on execution of a first application installed in the electronic device.

The at least one instruction according to one embodiment may cause, when executed by the at least one processor, the electronic device to stop execution of at least one application among other applications installed in the electronic device when re-measurement of the first biometric information is started.

The at least one instruction according to one embodiment may cause, when executed by the at least one processor, the electronic device to acquire the second biometric signal for a longer time than the time point of acquiring the first biometric signal.

The at least one instruction according to one embodiment may cause, when executed by the at least one processor, the electronic device to operate the first sensor at a driving frequency greater than a driving frequency of the first sensor for acquiring the first biometric signal.

The at least one instruction according to one embodiment may cause, when executed by the at least one processor, the electronic device to acquire the second biometric signal using light output with an intensity greater than an intensity of light output from the first sensor used to acquire the first biometric signal.

The electronic device according to one embodiment may include a display.

The at least one instruction according to one embodiment may cause, when executed by the at least one processor, the electronic device to determine whether re-measurement of the second biometric information different from the first biometric information is requested within a designated time from the time point of acquiring the second data corresponding to the second biometric signal, when re-measurement of the second biometric information is requested.

The at least one instruction according to one embodiment may cause, when executed by the at least one processor, the electronic device to display a first measurement result for the first biometric information and a second measurement result for the second biometric information on the display, when measurement of the second biometric information is requested within the designated time from the time point of acquiring the second data.

An operation method of an electronic device according to one embodiment may include acquiring a first biometric signal for measuring first biometric information of a user based on a first configuration value through a first sensor included in the electronic device.

The operation method of the electronic device according to one embodiment may include acquiring first data on the first biometric information measured based on the first biometric signal.

The operation method of the electronic device according to one embodiment may include determining whether re-measurement of the first biometric information is requested within a designated time from the time point of acquiring the first data, when re-measurement of the first biometric information is requested.

The operation method of the electronic device according to one embodiment may include acquiring a second biometric signal for measuring the first biometric information based on a second configuration value different from the first configuration value through the first sensor based on the determination that re-measurement of the first biometric information is requested within the designated time from the time point of acquiring the first data.

The operation method of the electronic device according to one embodiment may include identifying a measurement state of the first biometric information using the first sensor of a second sensor included in the electronic device.

The operation method of the electronic device according to one embodiment may include acquiring the second biometric signal according to a result of the identifying.

The operation method of the electronic device according to one embodiment may include identifying a contact state of the user's skin with the electronic device using the first sensor.

The operation method of the electronic device according to one embodiment may include preventing the second biometric signal from being acquired when the contact state of the user's skin does not satisfy a predetermined condition.

The operation method of the electronic device according to one embodiment may include identifying a movement of the electronic device using the second sensor.

The operation method of the electronic device according to one embodiment may include preventing the second biometric signal from being acquired when the movement of the electronic device does not satisfy the predetermined condition.

The operation method of the electronic device according to one embodiment may include stopping transmission or reception of a signal that affects the quality of the second biometric signal based on the determining that re-measurement of the first biometric information is requested within the designated time from the time point of acquiring the first data.

The operation method of the electronic device according to one embodiment may include starting re-measurement of the first biometric information of the user based on execution of a first application installed in the electronic device.

The operation method of the electronic device according to one embodiment may include stopping execution of at least one application among other applications installed in the electronic device when re-measurement of the first biometric information is started.

The operation method of the electronic device according to one embodiment may include operating the first sensor at a driving frequency greater than a driving frequency of the first sensor for acquiring the first biometric signal.

The operation method of the electronic device according to one embodiment may include measuring the second biometric signal using light output with an intensity greater than an intensity of light output from the first sensor used to acquire the first biometric signal.

The operation method of the electronic device according to one embodiment may include determining whether re-measurement of the second biometric information different from the first biometric information is requested within a designated time from the time point of acquiring the second data corresponding to the second biometric signal, when re-measurement of the second biometric information is requested.

The operation method of the electronic device according to one embodiment may include displaying a first measurement result for the first biometric information and a second measurement result for the second biometric information on the display, when measurement of the second biometric information is requested within the designated time from the time point of acquiring the second data.

A non-transitory recording medium according to one embodiment may store at least one instruction capable of executing an operation of acquiring a first biometric signal for measuring first biometric information of a user based on a first configuration value through a first sensor included in an electronic device.

The non-transitory recording medium according to one embodiment may store at least one instruction capable of executing an operation of acquiring first data on the first biometric information measured based on the first biometric signal.

The non-transitory recording medium according to one embodiment may store at least one instruction capable of executing an operation of determining whether re-measurement of the first biometric information is requested within a designated time from the time point of acquiring the first data, when re-measurement of the first biometric information is requested.

The non-transitory recording medium according to one embodiment may store at least one instruction capable of executing an operation of acquiring a second biometric signal for measuring the first biometric information based on a second configuration value different from the first configuration value through the first sensor based on the determination that re-measurement of the first biometric information is requested within the designated time from the time point of acquiring the first data.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the present disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC). Various embodiments as set forth herein may be implemented as software (e.g., the program 140) including one or more instructions that are stored in a storage medium (e.g., internal memory 136 or external memory 138) that is readable by a machine (e.g., the electronic device 101, 401). For example, a processor (e.g., the processor 120) of the machine (e.g., the electronic device 101, 401) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore^{™}), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities, and some of the multiple entities may be separately disposed in different components. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

## Claims

1. An electronic device (101 of FIG. 1; 201 of FIG. 2) comprising:
a first sensor (176 of FIG. 1; 271 of FIG. 2);
memory (130 of FIG. 1; 230 of FIG. 2); and
at least one processor (120 of FIG. 1; 220 of FIG. 2),
wherein the memory stores at least one instruction that, when executed by the at least one processor, causes the electronic device to:
acquire a first biometric signal for measuring first biometric information of a user based on a first configuration value through the first sensor;
acquire first data on the first biometric information measured based on the first biometric signal;
determine whether re-measurement of the first biometric information is requested within a designated time from a time point of acquiring the first data when re-measurement of the first biometric information is requested; and
acquire a second biometric signal for measuring the first biometric information based on a second configuration value different from the first configuration value through the first sensor based on the determination that re-measurement of the first biometric information is requested within the designated time from the time point of acquiring the first data.

2. The electronic device of claim 1, further comprising:
a second sensor (176 of FIG. 1; 272 of FIG. 2),
wherein the at least one instruction further causes, when executed by the at least one processor, the electronic device to:
identify a measurement state of the first biometric information using the first sensor or the second sensor; and
acquire the second biometric signal according to the identification result.

3. The electronic device of claim 1 or claim 2, wherein the at least one instruction further causes, when executed by the at least one processor, the electronic device to:
identify a contact state of the user's skin with the electronic device using the first sensor; and
prevent the acquisition of the second biometric signal when the contact state of the user's skin does not satisfy a predetermined condition.

4. The electronic device of one of claim 1 to claim 3, wherein the at least one instruction further causes, when executed by the at least one processor, the electronic device to:
identify a movement of the electronic device using the second sensor; and
prevent the acquisition of the second biometric signal when the movement of the electronic device does not satisfy a predetermined condition.

5. The electronic device of one of claim 1 to claim 4, wherein the at least one instruction further causes, when executed by the at least one processor, the electronic device to stop transmission or reception of a signal that affects the quality of the second biometric signal based on the determination that re-measurement of the first biometric information is requested within the designated time from the time point of acquiring the first data.

6. The electronic device of one of claim 1 to claim 5, wherein the at least one instruction further causes, when executed by the at least one processor, the electronic device to:
start re-measurement of the first biometric information of the user based on execution of a first application installed in the electronic device; and
stop execution of at least one application among other applications installed in the electronic device when re-measurement of the first biometric information is started.

7. The electronic device of one of claim 1 to claim 6, wherein the at least one instruction further causes the electronic device to acquire the second biometric signal for a longer time than the time point of acquiring the first biometric signal, as at least a part of the operation of acquiring of the second biometric signal based on the second configuration value.

8. The electronic device of one of claim 1 to claim 7, wherein the at least one instruction causes the electronic device to operate the first sensor at a driving frequency greater than a driving frequency of the first sensor for acquiring the first biometric signal, as at least a part of the operation of acquiring the second biometric signal based on the second configuration value.

9. The electronic device of one of claim 1 to claim 8, wherein at least one instruction causes the electronic device to acquire the second biometric signal using light output with an intensity greater than an intensity of light output from the first sensor used to acquire the first biometric signal, as at least a part of the operation of acquiring the second biometric signal based on the second configuration value.

10. The electronic device of one of claim 1 to claim 9, further comprising;
a display (160 of FIG. 1; 260 of FIG. 2),
wherein the at least one instruction further causes, when executed by the at least one processor, the electronic device to:
determine whether re-measurement of the second biometric information different from the first biometric information is requested within a designated time from the time point of acquiring the second data corresponding to the second biometric signal, when re-measurement of the second biometric information is requested; and
display a first measurement result for the first biometric information and a second measurement result for the second biometric information on the display, when measurement of the second biometric information is requested within the designated time from the time point of acquiring the second data.

11. An operation method of an electronic device (101 of FIG. 1; 201 of FIG. 2), comprising:
acquiring a first biometric signal for measuring first biometric information of a user based on a first configuration value through a first sensor (176 of FIG. 1; 271 of FIG. 2) included in the electronic device;
acquiring first data on the first biometric information measured based on the first biometric signal;
determining whether re-measurement of the first biometric information is requested within a designated time from the time point of acquiring the first data, when re-measurement of the first biometric information is requested; and
acquiring a second biometric signal for measuring the first biometric information based on a second configuration value different from the first configuration value through the first sensor based on the determination that re-measurement of the first biometric information is requested within the designated time from the time point of acquiring the first data.

12. The operation method of claim 11, further comprising;
identifying a measurement state of the first biometric information using the first sensor of a second sensor (176 of FIG. 1; 272 of FIG. 2) included in the electronic device; and
acquiring the second biometric signal according to a result of the identifying.

13. The operation method of one of claim 11 and claim 12, further comprising:
identifying a contact state of the user's skin with the electronic device using the first sensor; and
preventing the second biometric signal from being acquired when the contact state of the user's skin does not satisfy a predetermined condition.

14. The operation method of one of claim 11 and claim 13, further comprising:
identifying a movement of the electronic device using the second sensor; and
preventing the second biometric signal from being acquired when the movement of the electronic device does not satisfy the predetermined condition.

15. A non-transitory recording medium which stores at least one instruction capable of executing operations of:
acquiring a first biometric signal for measuring first biometric information of a user based on a first configuration value through a first sensor (176 of FIG. 1; 271 of FIG. 2) included in an electronic device (101 of FIG. 1; 201 of FIG. 2);
acquiring first data on the first biometric information measured based on the first biometric signal;
determining whether re-measurement of the first biometric information is requested within a designated time from the time point of acquiring the first data, when re-measurement of the first biometric information is requested; and
acquiring a second biometric signal for measuring the first biometric information based on a second configuration value different from the first configuration value through the first sensor based on the determination that re-measurement of the first biometric information is requested within the designated time from the time point of acquiring the first data.
